Europäisches Patentamt

(19) European Patent Office

Office européen des brevets

(11) Publication number: **0 266 163**
**A2**

(12) **EUROPEAN PATENT APPLICATION**

(21) Application number: **87309458.5**

(22) Date of filing: **27.10.87**

(51) Int. Cl.⁴: **C 08 B 37/00**
**C 12 P 19/04**

(30) Priority: **28.10.86 US 923924**

(43) Date of publication of application:
**04.05.88 Bulletin 88/18**

(84) Designated Contracting States:
**AT BE CH DE ES FR GB GR IT LI LU NL SE**

(71) Applicant: **PFIZER INC.**
**235 East 42nd Street**
**New York, N.Y. 10017 (US)**

(72) Inventor: **Cannon, John Joseph**
**8, Pheasant Run Drive**
**Gales Ferry Connecticut (US)**

(74) Representative: **Moore, James William, Dr.**
**Pfizer Limited Ramsgate Road**
**Sandwich Kent CT13 9NJ (GB)**

(54) **Polysaccharide isolation process.**

(57) A process for the isolation of a polysaccharide from an aqueous solution such as a fermentation broth by contacting the solution with from about 2 to 20 weight percent of a polyethylene glycol of number molecular weight of at least about 1000, and separating the solids which precipitate. The process is adaptable to the precipitation of both nonionic polysaccharides such as scleroglucan and ionic polysaccharides such as xanthan and succinoglycan.

EP 0 266 163 A2

## Description

## POLYSACCHARIDE ISOLATION PROCESS

The present invention concerns a process for the isolation of polysaccharides from aqueous solutions such as fermentation broths.

Polysaccharides such as scleroglucan, xanthan and succinoglycan have received considerable attention in recent years, primarily because of the high viscosity of aqueous solutions of these biopolymers. Thus, the polysaccharides have been incorporated as thickening agents in such as human and animal food products, cosmetic formulations and water base paints. Of particular importance is their use as mobility control agents in oil recovery.

These biopolymers are normally produced by fermentation, and the fermentation broth is often used directly, either as is or separated from the microbial cells, and generally diluted.

Certain uses, however, require the isolation of the polysaccharide from other water-soluble constituents of the broth. In this case, a current procedure treats the broth or its filtrate with about 0.5 volume or more of a water-soluble organic solvent such as a lower alcohol or ketone to flocculate the polysaccharide, and then filters or centrifuges the floc from the aqueous medium. Such isolation, disclosed for example in U.S. Patents 3,301,848, 3,436,311 and 4,326,053, results in a polysaccharide of high purity in good yield. But the large volume of solvent required adds considerably to the material and processing cost for the isolation.

Other biopolymer isolation procedures are based on ultrafiltration or salt precipitation methods. Xanthan gum, for example, can be obtained as a concentrated aqueous stream by filtration and ultrafiltration of a xanthan-containing fermentation broth, as disclosed in U.S. Patent 4,299,825. The concentrate obtained, however, still contains 85 to 90 percent water and does not offer the handling and processing advantages of an actual precipitate. Salt precipitation methods include, for example, quaternary amine salt precipitation (U.S. Patent 3,119,812); aluminum salt precipitation (U.S. Patent 4,051,317); amine salt precipitation (U.S. Patent 4,254,257); alkaline precipitation in the presence of divalent cations (U.S. Patent 3,382,229); and recovery with trivalent metal ions (Japanese Kokai 196009/84). While such precipitation methods can offer excellent recovery yields, the biopolymer is obtained as a water-insoluble salt. Removal of the complexing agent, for example, quaternary amine or divalent cation, must therefore precede solubilization of the biopolymer, and involves further processing and material costs.

A need therefore exists for a simpler, more efficient method of isolating the polysaccharide from fermentation broths and other aqueous solutions of the polysaccharide. The primary objective of the present invention is to provide such a method.

It has now been found that the addition of a small amount of a polyethylene glycol to a fermentation broth or other polysaccharide solution causes the polysaccharide to readily precipitate from solution in a form which is easily filtered or centrifuged free of the solution.

The present invention is therefore directed to a process for the isolation of a polysaccharide from an aqueous solution of the polysaccharide, which comprises contacting the solution with from about 2 to 20 weight percent of a polyethylene glycol having a number average molecular weight of at least about 1000, and separating the solids which precipitate.

The solution is preferably in the form of a fermentation broth, especially a broth essentially free of microbial cells, and the polysaccharide is preferably present in the solution at a concentration of from about 2000 to 40,000 ppm. The polysaccharide may be a nonionic polysaccharide such as scleroglucan, or an ionic polysaccharide such as xanthan or succinoglycan. With nonionic polysaccharides, the polyethylene glycol preferably also is nonionic. With ionic polysaccharides, the polyethylene glycol preferably also is ionic, and the solution may contain dissolved inorganic salt and/or be adjusted to a pH of less than about 2 to enhance the precipitation. The precipitated solids are preferably separated from the solution by centrifugation.

The present isolation process is applicable to any aqueous polysaccharide solution, but is particularly suitable for fermentation broths in which the polysaccharide is produced. The polysaccharide may be precipitated directly from the whole broth, in which case it will be admixed with microbial cells from the broth, or the polysaccharide may be precipitated from a broth which has been essentially freed of the microbial cells by such as filtration or centrifugation.

In either case, the polysaccharide concentration will normally be from about 200 to 50,000 ppm by weight of the solution. Concentrations much below 200 ppm are too low for effective recovery of the polysaccharide, while solutions with polysaccharide concentrations above 50,000 are too viscous to easily process. Preferably, the polysaccharide concentration is from about 2000 to 40,000 ppm.

The polysaccharide may be either nonionic or ionic. Suitable nonionic polysaccharides include, for example, scleroglucan, pullulan, pendulan and schizophyllan, while the ionic polysaccharides include such as xanthan, succinoglycan, whelan gum, rhamsan gum and chitosan.

The nonionic polysaccharide, such as scleroglucan, is precipitated directly from the broth at its natural pH, which is normally from about 5 to 7, by the addition of a nonionic polyethylene glycol, i.e., a standard, uncharged, ethylene glycol condensation polymer, of weight molecular weight of at least about 1000 in an amount of from about 2 to 20 weight percent of the solution. Amounts much less than 2 percent result in incomplete precipitation of the polysaccharide, while amounts greater than 20 percent offer no beneficial effect. The normal glycol addition is from about 6 to 15 weight percent of the solution. The polysaccharide precipitation occurs within minutes of the addition. The resulting slurry is stirred an additional short period and the polysaccharide precipitate is separated by such as filtration or centrifugation, the latter being preferred. The precipitate may or may not be washed with a suitable solvent such as methanol or acetone, then dried or used as is without drying.

The ionic polysaccharide, however, does not ordinarily precipitate under such conditions. With this type of polysaccharide, use of a nonionic ethylene glycol requires an additional treatment of the solution. Some ionic polysaccharides such as xanthan precipitate if the solution pH is adjusted to below about 2. Such pH adjustment can have a denaturing effect on the biopolymer, however, resulting in a loss of desired rheological properties. Other ionic polysaccharides such as rhamsan gum precipitate when an inorganic salt such as sodium chloride or ammonium sulfate is added along with the nonionic polyethylene glycol, the salt addition normally being in the amount of from 1 to 5 weight percent of the solution.

Preferably, however, the ionic poysaccharide is precipitated at its natural pH by the addition of an ionic polyethylene glycol, the molecular weight and addition requirements being essentially the same as indicated for the nonionic polyethylene glycol addition. Such ionic polyethylene glycols may be cationic, with a positive charge, or anionic, with a negative charge, and are available from Union Carbide Corporation, Danbury, Ct. Thus, with anionic polysaccharides such as xanthan and succinoglycan, an anionic polyethylene glycol is used, while with cationic polysaccharides such as chitosan, a cationic polyethylene glycol is used.

Such use of the ionic polyethylene glycol is highly advantageous, since it avoids the problem of possibly denaturing the polysaccharide by pH adjustment. Inorganic salt addition at the levels previously indicated may be used with the ionic polyethylene glycol, however, to enhance precipitation without denaturing the polysaccharide.

The following examples are merely illustrative, and are not to be construed as limiting the present process, the scope of which is defined by the claims.

## Example 1

### Scleroglucan Isolation from Cell-free Broth

Some 10 g of polyethylene glycol (PEG) 2000(1) was added with stirring to 100 g of homogenized, cell-free S. rolfsii broth prepared as in U.S. Patent 3,301,848, having an initial viscosity of 900 cp and containing 5000 ppm (5.0 g/liter) scleroglucan. A fibrous precipitate started to form after several minutes of stirring, and the precipitation appeared complete within 30 minutes. The resulting slurry was centrifuged for 10 minutes at 6000 × g to yield 4.4 g of wet precipitate and a centrifugate of 2.2 cp viscosity, indicating essentially 100 percent removal of the polysaccharide from the broth.

Rheological assays indicated no change in the viscosity effectiveness and thermal activity of the resolubilized scleroglucan biopolymer.

Repetition of the precipitation with the addition of the PEG 2000 at a level of 3 weight percent of the broth produced a similar result.

(1) Polyglycol E 2000; Dow Chemical USA, Midland, MI

## Example 2

### Scleroglucan Isolation from Whole Broth

To 300 g of a S. rolfsii whole broth fermented as in Example 1 and containing 13,000 ppm (13.1 g/liter) scleroglucan was added 30g PEG 4000(1) (10 weight percent). The mixture was stirred 30 minutes and then centrifuged at 5000 × g for 15 minutes to yield 170 g wet solids. Further centrifugation at 12,000 × g for 20 minutes reduced the wet solids weight to 82 g. The scleroglucan content of the final solids was 47,700 ppm, indicating a 3.6 fold concentration of the biopolymer from whole broth.

Similar results were obtained with a PEG 4000 level of 20 weight percent of the broth.

(1) Polyglycol E 4000; Dow Chemical USA

## Example 3

### Whelan Gum Isolation from Cell-free Broth

Some 100 g of an Alcaligenes (ATCC 31555) whole broth fermented as in British Disclosed Patent Application GB2058107A, having a viscosity of 8500 cp and containing 9800 ppm whelan gum (9.8 g/liter) was diluted 4 fold with deionized water. The diluted broth, at a viscosity of 1500 cp, was centrifuged at 12,000 × g for 20 minutes to separate the microbial cells in the broth from the biopolymer solution.

The biopolymer solution was adjusted to pH 1.0 with 1 molar sulfuric acid, and sodium chloride (2 g/100 g solution) was added. Then PEG 8000(1)(10 weight percent) was added, and the solution was stirred 20 minutes. The resulting slurry was centrifuged at 12,000 × g for 20 minutes. The wet centrifuge solids were dried under vacuum at 50°C to yield 0.57 g dry solids/100 g broth.

(1) Polyglycol E 8000; Dow Chemical USA

## Example 4

### Rhamsan Gum Isolation from Cell-free Broth

An Alcaligenes (ATCC 3965) fermentation broth fermented as in British Disclosed Patent Application GB2058107A having a viscosity of 2480 cp and containing 5100 ppm rhamsan gum (5.1 g/liter) was processed as in Example 3 except the broth was diluted 3 fold to give a dilute broth with viscosity of 980 cp; the centrifuged broth pH was not adjusted; and the precipitating agent was PEG 8000 (5 weight percent) plus anionic-PEG 8000(1) (5 weight percent). The dried solids weighed 0.86 g/100 g broth.

(1) Experimental MLX 1185 Modified Polyalkylene Glycol; Union Carbide Corp, Danbury, CT

## Example 5

### Xanthan Isolation from Whole Broth

Some 15 g of anionic-PEG 8000 and 2 g sodium chloride were added to 100 g whole Xanthomonas campestris broth fermented as in U.S. Patent 4,119,546, having a viscosity of 7300 cp and containing 33,600 ppm (33.6 g/liter) xanthan biopolymer. The treated broth was stirred vigorously for one hour, and the resulting suspension was centrifuged at 6000 × g for 10 minutes to yield 13.9 g wet centrifuge cake.

Resuspension of the wet cake in wet saline indicated that the precipitated polymer retained its original viscosity effectiveness and thermal activity. The wet cake may also be dried without affecting its rheological properties.

Substitution of PEG 8000 for anionic-PEG 8000 in the above process required adjustment of the broth to a pH of 1.5 to effect precipitation.

Example 6

Succinoglycan Isolation from Whole Broth

To 200 g succinoglycan whole broth fermented as disclosed in J. Gen. Microbiology, 102, 13-21 (1977) and containing 17,900 ppm (17.9 g/liter) succinoglycan biopolymer was added 30 g anionic-PEG 8000. Vigorous stirring for one hour produced no visible precipitation, which was finally effected by the addition of 4 g of sodium chloride. The resulting slurry was centrifuged at 6000 × g for 10 minutes to yield 52.1 g wet centrifuge cake.

Substitution of PEG 8000 for anionic-PEG 8000 in the above procedure produced no precipitation, even with the salt addition.

## Claims

1. A process for the isolation of a polysaccharide from an aqueous solution of the polysaccharide, which comprises contacting the solution with from about 2 to 20 weight percent of a polyethylene glycol having a number average molecular weight of at least about 1000, and separating the solids which precipitate.

2. The process of claim 1 wherein the solution is in the form of a fermentation broth.

3. The process of claim 2 wherein the fermentation broth is essentially free of microbial cells.

4. The process of claim 1 wherein the polysaccharide is present in the solution at a concentration of from about 2000 to 40,000 ppm.

5. The process of claim 1 wherein both the polysaccharide and the polyethylene glycol are nonionic.

6. The process of claim 5 wherein the nonionic polysaccharide is scleroglucan.

7. The process of claim 1 wherein the polysaccharide is an ionic polysaccharide.

8. The process of claim 7 wherein the ionic polysaccharide is xanthan or succinoglycan.

9. The process of claim 7 wherein both the polysaccharide and the polyethylene glycol are ionic.

10. The process of claim 7 wherein the solution contains a dissolved inorganic salt.

11. The process of claim 7 wherein the solution is adjusted to a pH of less than about 2.

12. The process of claim 1 wherein the precipitated solids are separated from the solution by centrifugation.